# EUROPÄISCHE PATENTSCHRIFT

(11) **EP 1 196 427 B1**
(45) Veröffentlichungstag und Bekanntmachung des Hinweises auf die Patenterteilung: **23.05.2007**
(21) Anmeldenummer: 00954359.6
(22) Anmeldetag: 17.07.2000
(51) Int. Cl.: C07J 1/00, A61K 31/565, G01N 33/60, C07J 51/00, C07J 53/00, C07J 21/00, C07J 71/00, C07J 43/00, A61P 5/36, A61P 5/32

(54) **C-19-HALOGENSUBSTITUIERTE, 5-SUBSTITUIERTE ODER 6,10-CARBOZYKLISCH-KONDENSIERTE STEROIDE DER ANDROST-9(11)-EN-REIHE, VERFAHREN ZU IHRER HERSTELLUNG SOWIE IHRE VERWENDUNG**
C-19-HALOGEN-SUBSTITUTED STEROIDS OF THE ANDROST-9(11)-ENE-SERIES, METHODS FOR THE PRODUCTION AND USE THEREOF
STEROIDES SUBSTITUES PAR HALOGENE EN C-19 DE LA SERIE ANDROST-9(11)-ENE, PROCEDES PERMETTANT DE LES PREPARER ET LEUR UTILISATION

(30) Priorität: 15.07.1999 DE 19934088
(43) Veröffentlichungstag der Anmeldung: 17.04.2002
(73) Patentinhaber: Bayer Schering Pharma Aktiengesellschaft, 13353 Berlin (DE)
(72) Erfinder: NEEF, Günter, 10711 Berlin (DE); GOLDE, Roland, 16562 Bergfelde (DE); FRITZEMEIER, Karl-Heinrich, 13505 Berlin (DE)
(86) Internationale Anmeldenummer: PCT/DE2000/002390
(87) Internationale Veröffentlichungsnummer: WO 2001/005805

(56) Entgegenhaltungen:
- US-A- 3 101 356
- US-A- 5 218 110
- US-A- 5 318 961
- US-A- 5 436 237
- D. R. HERBST ET AL: "TOTALLY SYNTHETIC STEROID HORMONES. XVIII. dl-17.BETA.-HYDROXY-18-METHYLANDROSTA-4,9( 11)-DIEN-3-ONE AND dl-18-METHYL-5.ALPHA.-ANDROSTAN-3,17-DIONE " STEROIDS., Bd. 11, Nr. 6, Juni 1968 (1968-06), Seiten 935-943, XP002154398 ELSEVIER SCIENCE PUBLISHERS, NEW YORK, NY., US ISSN: 0039-128X
- SCHMIT J-P ET AL: "THE ANDROGEN RECEPTOR BINDING SITE A CONFORMATIONAL STUDY OF STEROIDS IN RELATION WITH THEIR AFFINITY 1. INTERACTION WITH THE D RING" JOURNAL OF STEROID BIOCHEMISTRY, Bd. 13, Nr. 12, 1980, Seiten 1387-1394, XP002154399 ISSN: 0022-4731
- KIRCHHOFF J ET AL: "DIFFERENCES IN THE STEROID BINDING SITE SPECIFICITIES OF RAT PROSTATE ANDROGEN RECEPTOR AND EPIDIDYMAL ANDROGEN BINDING PROTEIN" JOURNAL OF STEROID BIOCHEMISTRY, Bd. 10, Nr. 5, 1979, Seiten 487-498, XP002154400 ISSN: 0022-4731
- G. NEEF ET AL: "A radical approach to the synthesis of 9(10->19)abeo-steroids" TETRAHEDRON., Bd. 49, Nr. 4, 22. Januar 1993 (1993-01-22), Seiten 833-840, XP002154401 ELSEVIER SCIENCE PUBLISHERS, AMSTERDAM., NL ISSN: 0040-4020 in der Anmeldung erwähnt
- YATES, JEAN ET AL: "Inhibitors of human adrenal C17-20 lyase and C19-5-ene, 3.beta.-hydroxysteroid dehydrogenase" J. STEROID BIOCHEM. (1975), 6(9), 1325-7 , XP002163772
- GOLDMAN A S ET AL: "EFFECTS OF NEW MULTI-SITE HORMONE BLOCKERS ON THE FERTILITY OF MALE RATS" JOURNAL OF ENDOCRINOLOGY,GB,BRISTOL, Bd. 69, Nr. 1, 1. April 1976 (1976-04-01), Seiten 11-21, XP000645942 ISSN: 0022-0795
- CHEMICAL ABSTRACTS, vol. 61, no. 4, 17. August 1964 (1964-08-17) Columbus, Ohio, US; abstract no. 4665h, F. A. KINCL ET AL: "Pituitary gonadotropin inhibitory action of neutral steroids" XP002163776 & ACTA ENDOCRINOL., Bd. 46, Nr. 2, 1964, Seiten 300-306,
- DATABASE EPODOC [Online] EUROPEAN PATENT OFFICE, THE HAGUE, NL; XP002163778 & CN 1 174 843 A (SHANGHAI INST ORGANIC CHEM) 4. März 1998 (1998-03-04) -& CHEMICAL ABSTRACTS, vol. 132, no. 15, 10. April 2000 (2000-04-10) Columbus, Ohio, US; abstract no. 194549, Seite 616; Spalte 1; XP002163777 -& "Chemical Abstracts Formula Index, C15H23N3OS - C28H37Br3O18, Volume 132" 2000 , CHEMICAL ABSTRACTS , COLUMBUS OHIO, US XP002163775
- LESUISSE, DOMINIQUE ET AL: "Structure-Activity Relationships of a New Family of Steroidal Aromatase Inhibitors. 1. Synthesis and Evaluation of a Series of Analogs Related to 19-[(Methylthio)methyl]androstenedione (RU54115)" J. MED. CHEM. (1996), 39(3), 757-72 , XP002163773
- GUARNA ANTONIO ET AL: "19-Nor-10-azasteroids: A novel class of inhibitors for human steroid 5-alpha-reductases 1 and 2." JOURNAL OF MEDICINAL CHEMISTRY, Bd. 40, Nr. 7, 1997, Seiten 1112-1129, XP002163774 ISSN: 0022-2623

## Beschreibung

Root et al beschreiben in J. Endocrinol. 1976, 69, 11-21 Antiandrogene mit einer 5β-CN-Gruppe.

Die vorliegende Erfindung betrifft die Verbindungen der allgemeinen Formel II 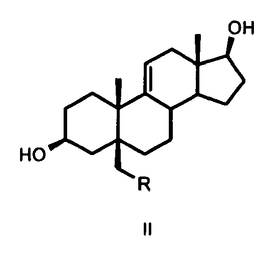 mit einem Rest R in der Bedeutung von:
R = -(CH₂)ₙ-CH₂-R¹, -(CH₂)ₙ-CH₂-OR¹, -(CH₂)ₙ-CH₂-OCOR¹, -(CH₂)ₙ-CH₂-SR¹,
-(CH₂)ₙ-CH₂-NR¹R², -(CH₂)ₙ-CHO, -(CH₂)ₙ-CN
worin n die Werte von 0-5 annehmen kann und die Reste R¹ und R² unabhängig voneinander für Wasserstoff oder einen geradkettigen oder verzweigten, gesättigten oder ungesättigten Kohlenwasserstoffrest mit bis zu 18 C-Atomen stehen, wobei dieser Rest gegebenfalls zusätzliche funktionelle Gruppen und carbocyclische oder heterocyclische Ringelemente enthalten kann.

Die Silylierung der 17β-Hydroxy-Gruppe der C-19-halogensubstituierten Steroide der Androst-9(11)-en-Reihe der allgemeinen Formel I 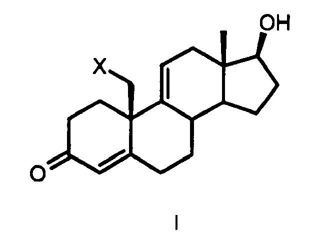 in der X einen Halogenrest, ausgewählt aus Br, I bedeutet und führt zu einem 17β-Silylether der allgemeinen Formel la 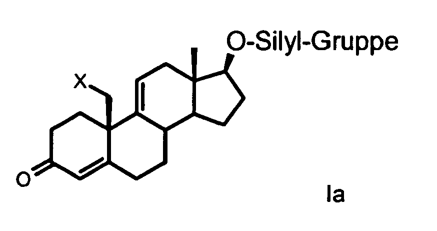 in der X = Halogen, ausgewählt aus Br, I bedeutet und der ein wichtiges Zwischenprodukt für die weitere Synthese in einem sogenannten Tandemprozess zu den neuen Verbindungen der allgemeinen Formel II darstellt.

So führt, am Beispiel des 17β-(tert.-Butyltrimethylsilyloxy)-19-iod-androsta-4,9(11)-dien-3-ons 8 dargestellt, die Umsetzung mit Mercaptoessigsäuremethylester in Gegenwart einer geeigneten Base zur Bildung eines thia-verbrückten Derivats 9. Ausgangsprodukte können ebenso die anderen 17β-silylierten C-19-Halogenderivate sein.

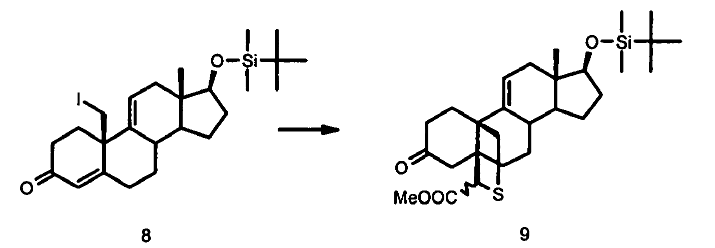

Auf diese Weise wird die funktionelle Gruppe an C-19 genutzt, um eine C-C-Verknüpfung mit der tertiären Position C-5 zu erreichen. Bekanntermaßen ist die stereoselektive Einführung funktioneller Gruppen in die tertiären Positionen des Steroidgerüsts ein Problem der präparativen Chemie, für das generelle Lösungen nicht zur Verfügung stehen. So ist zwar die Einführung einer 5β-Methylgruppe durch Umsetzung von Testosteron mit metallorganischen Reagenzien bekannt ( z.B. C. Petrier et al., *Tetrahedron Lett. 25*, 3463, 1984), eignet sich jedoch nicht zur Einführung höherer Alkylsubstituenten oder funktionell substituierter Alkylgruppen.

Das thia-verbrückte Derivat 9 wird dann zu Verbindungen der allgemeinen Formel II umgesetzt.
Nach den in der Literatur beschriebenen Erfahrungen aus der Normalreihe (9(11)-gesättigt) ist das Resultat der Umsetzung des silylierten Halogenids la, z.B. des lodids 8 mit Mercaptoessigsäuremethylester nicht vorhersehbar gewesen. Wie von Halpern et al. *(Steroids 4,* 1-30, 1964), Santaniello and Caspi *(J.Steroid Biochem. 7*, 223-227, 1976) und Wright et al. *(J.Chem.Soc. Perkin Trans. I,* 1989, 1647-1655) beschrieben, ist die nukleophile Substitution an C-19 in Gegenwart des 3-Oxo-4-en-Strukturelements äußerst erschwert und führt vorwiegend zu Gerüstumlagerungen. Umso überraschender ist der glatte Verlauf der Reaktion einer Verbindung der Formel la → thia-verbrücktes Derivat 9, der mechanistisch als nukleophiler Halogen-Schwefelaustausch mit nachfolgender Michael-Addition zu deuten ist (Tandemprozeß).

Schema 1 verdeutlicht einen beispielhaften Syntheseweg:

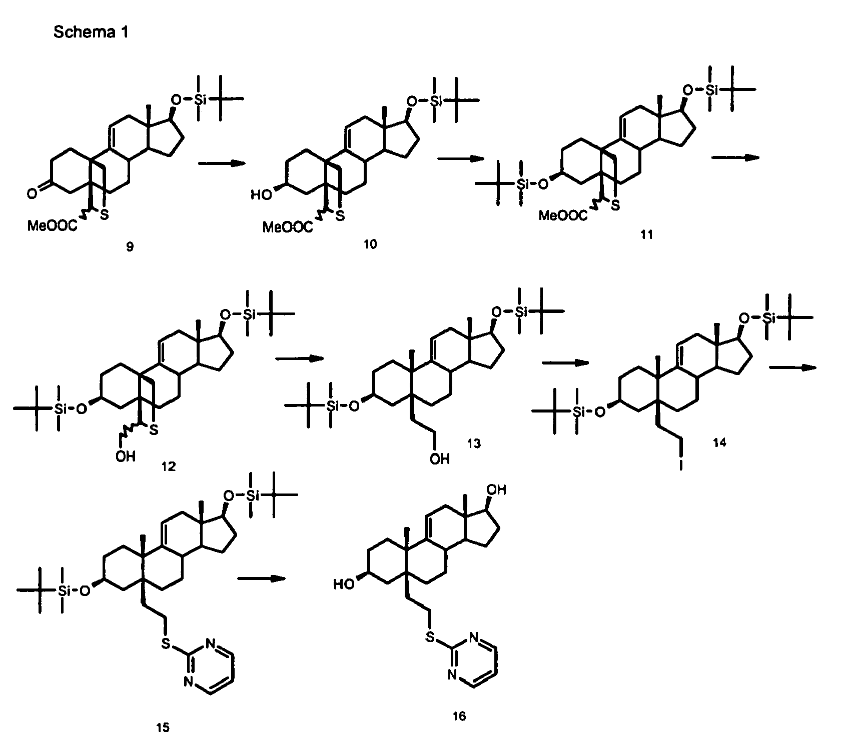

Das durch Schema 1 beschriebene Verfahren bietet eine Reihe von Möglichkeiten, Derivate der allgemeinen Formel II herzustellen. Es ist offensichtlich, daß z. B. die Zwischenprodukte 13 und 14 eine Fülle von Verbindungen zur Herstellung solch neuer Steroide ergeben.

Die Verbindungen der allgemeinen Formel II sind eine neue Klasse antiandrogen wirksamer Steroide und eignen sich damit zur Behandlung androgen-abhängiger Erkrankungen (Prostatakarzinom, Prostatahyperplasie).

Gegenstand der Erfindung sind neben den Verbindungen der allgemeinen Formel II gemäß Anspruch 1, ihre Verwendung gemäß Anspruch 2, sowie diese enthaltende pharmazeutische Mittel gemäß Anspruch 3, die als aktiven Bestandteil mindestens eine Verbindung der allgemeinen Formel II enthalten.

Die nachfolgenden Beispiele sollen die Erfindung näher erläutern, ohne sie darauf zu beschränken.

### Herstellung der Ausgangsverbindungen

### Beispiel A: 17-β-Hydroxy-19-iod-androsta-4,9(11)-dien-3-on

### a. 17β-(tert.-Butyldimethylsilyloxy)-3,3-(2,2-dimethyl-trimethylendioxy)-1 Oβ-formyl-androst-9(11)-en-5α-ol (2)

Eine Lösung von 5,0 g (12,4 mmol) 3,3-(2,2-Dimethyl-trimethylendioxy)-10β-formyl-androst-9(11)-en-5α,17β-diol wird nach Zusatz von 3,43 g (50,4 mmol) Imidazol und 4,46 ml (14,7 mmol) einer 3,3M Lösung von tert.-Butyldimethylchlorsilan in Hexan 16 h bei Raumtemperatur gerührt. Zur Aufarbeitung verdünnt man mit Wasser und extrahiert mit Ethylacetat. Nach Chromatographie an Kieselgel mit Hexan/Ethylacetat 1:9 erhält man 5,60 g (87,0% d. Th.) des Silylethers vom Schmp. 168-170° C (Hexan), [α]_{D} -179,3° (CHCl₃, c = 0,5). ¹H-NMR (CDCl₃, 300 MHz): δ = 0,61 ppm (s,3H,H-18); 0,87 (s,9H,Si-tBu); 0,93 u. 0,94 (2s,je 3H,ketal-Me); 3,63 (t,J = 8 Hz, 1H,H-17); 4,45 (s,1H,OH); 5,62 (m,1H,H-11); 9,07 (s,1H,CHO).

### b. 17β-(tert.-Butyldimethylsilyloxy)-3,3-(2,2-dimethyl-trimethylendioxy)-androst-9(11)-en-5α,19-diol (3)

Eine Lösung von 2,58 g (4,97 mmol) des unter a. erhaltenen Produkts in 26 ml THF und 26 ml Methanol wird bei 0° C mit 211 mg (5,57 mmol) Natriumborhydrid versetzt und 1,5 h bei 0° C gerührt. Nach erneuter Zugabe von 105 mg (2,78 mmol) NaBH₄ wird weitere 75 min bei 0° C gerührt, die Reaktionsmischung anschließend in Eiswasser eingerührt und mit Ethylacetat extrahiert. Das kristalline Rohprodukt (2,46 g, 95% d. Th.) wird ohne weitere Reinigung in die Folgestufe eingesetzt.¹H-NMR (CDCl₃, 300 MHz): δ = 0,67 ppm (s,3H,H-18); 0,87 (s,9H,Si-tBu); 0,93 u. 0,99 (2s,je 3H,Ketal-Me); 3,41-3,73 (m,7H,CH₂OH, CH₂O, H-17); 4,51 (s,1 H, 5α-OH); 5,45 (d,J = 7,5 Hz,1H,H-11).

### c. 17β-(tert.-Butyldimethylsilyloxy)-3,3-(2,2-dimethyl-trimethylendioxy)-19-iod-androst-9(11)-en-5α-ol (4)

Zu einer Lösung von 16,07 g (30,9 mmol) des nach Beispiel Ab hergestellten Alkohols in 225 ml THF gibt man bei Raumtemperatur 20,72 g (79,0 mmol) Triphenylphosphin und 5,38 g (79,0 mmol) Imidazol. Unter Eiswasserkühlung fügt man dann portionsweise über ca. 5 min 10,03 g (39,5 mmol) lod zur Reaktionsmischung und rührt anschließend 1,5 h bei Umgebungstemperatur (23° C). Zur Aufarbeitung gießt man die Reaktionslösung in ca. 2 I einer 5-proz. wäßrigen Natriumthiosulfatlösung, die auf +5° C gekühlt wurde und extrahiert mit Ethylacetat. Nach Chromatographie des Rohprodukts an Kieselgel mit Hexan/Ethylacetat 9:1 erhält man 17,0 g (87,2% d. Th.) des lodids als farbloses Öl. ¹H-NMR (CDCl₃, 300 MHz): δ = 0,80 ppm (s,3H,H-18); 0,88 (s,9H,Si-tBu); 0,92 u. 2,00 (2s,je 3H,Ketal-Me); 3,41 - 3,74 (m,7H,CH₂l, CH₂O, H-17); 4,50 (s,1H, 5α-OH); 5,30 (d,J = 7,5 Hz,1H,H-11).

### d. 17β-(tert.-Butyldimethylsilyloxy)-3,3-(2,2-dimethyl-trimethylendioxy)-19-iod-androsta-5,9(11)-dien (5a) und 17β-(tert.-Butyldimethylsilyloxy)-3,3-(2,2-dimethyltrimethylendioxy)-19-iod-androsta-4,9(11)-dien (5b)

Das unter Beispiel Ac erhaltene Produkt (16,95 g, 26,9 mmol) wird in 85 ml Pyridin gelöst. Unter Eiswasserkühlung tropft man über ca. 15 min 3,91 ml (53,8 mmol) Thionylchlorid hinzu und rührt 45 min unter Eiswasserkühlung nach. Die so entstandene gelbe Suspension wird in ca. 1 I eines Gemisches aus gesättigter Kochsalzlösung (500 ml) und gesättigter NaHCO₃-Lösung eingerührt und mit Ethylacetat extrahiert.

Das nach Trocknung der EE-Extrakte über Na₂SO₄ und dem Einengen erhaltene Rohprodukt wird zur Entfernung von Pyridinresten mehrfach in Toluol aufgenommen und im Vakuum eingengt. Auf diese Weise erhält man 14,85 g eines Rohgemisches der isomeren Dehydratisierungsprodukte, das ohne weitere Reinigung zur Folgereaktion verwendet wird.

### e. 17β-Hydroxy-19-iod-androsta-4,9(11)-dien-3-on (6)

Eine Lösung von 14,85 g (24,3 mmol) des unter Ad erhaltenen Isomerengemisches in 325 ml Dichlormethan und 32 ml Wasser wird nach Zugabe von 64,6 ml (870 mmol) Trifluoressigsäure 4 h bei Raumtemperatur gerührt. Anschließend wird mit 200 ml Dichlormethan verdünnt, mit gesättigter Kochsalzlösung und NaHCO₃-Lösung gewaschen, über Natriumsulfat getrocknet und eingeengt. Das Rohprodukt wird über Kieselgel mit Hexan/Ethylacetat 1:1 chromatographiert und liefert nach Umkristallisation des Hauptprodukts aus Diisopropylether/Ethylacetat 6,62 g (66,1% d. Th.) der Titelverbindung vom Smp. 146° C (Zers.), [α]_{D} -2,0° (CHCl₃, c = 0,510). ¹H-NMR (CDCl₃, 300 MHz): δ = 0,87 ppm (s,3H,H-18); 3,56 (AB-q,J = 12 u. 4 Hz,2H,H-19); 3,78 (t,J = 9 Hz,1H,H-17); 5,60 (d,J = 7,5 Hz,1H,H-11); 5,85 (d,J = 1,5 Hz,1H,H-4).

### Beispiel B: 19-Brom-17β-hydroxy-androsta-4,9(11)-dien-3-on

In Analogie zur Verfahrensweise gemäß Beispiel A erhält man bei Verwendung von elementarem Brom anstelle von lod auf der Stufe Ac nach Wasserabspaltung (analog Beispiel Ad) und Säurebehandlung (Beispiel Ae) die Titelverbindung vom Schmp. 149° C (Zers.), [α]_{D} +20,4° (CHCl₃, c = 0,509). ¹H-NMR (CDCl₃, 300 MHz): δ = 0,80 ppm (s,3H,H-18); 3,64 (s,2H,H-19); 3,77 (t,J = 8 Hz,1H,H-17); 5,64 (d,J = 7,5 Hz,1H,H-11); 5,89 (d,J = 1 Hz,1 H,H-4).

### Beispiel C : 17β-(tert.-Butyldimethylsilyloxy)-3-oxo-2'H,5'H-thieno[3',4':5,10]-5β-estr-9(11)-en-2'ξ-carbonsäuremethylester

### a. 17β-(tert.-Butyldimethylsilyloxy)-19-iod-androsta-4,9(11)-dien-3-on (8)

Eine Reaktionslösung, bestehend aus 7,36 g (17,9 mmol) 17β-Hydroxy-19-iod-androsta-4,9(11)-dien-3-on, 7,48 g (110 mmol) Imidazol und 9,72 ml (32,1 mmol) tert.-Butyldimethylchlorsilan (3,3M in Hexan) in 40 ml DMF wird 16 h bei Raumtemperatur gerührt und wie üblich (Beispiel Aa) aufgearbeitet.

Man erhält 8,95 g (95% d. Th.) des Silylethers. ¹H-NMR (CDCl₃, 300 MHz): δ = 0,82 ppm (s,3H,H-18); 0,90 (s,9H,Si-tBu); 3,57 (t,J = 11 Hz,2H,H-19); 3,68 (t,J = 9 Hz,1H,H-17); 5,59 (d,J = 7,5 Hz, 1H, H-11); 5,85 (d,J = 1,5 Hz,1H,H-4).

### b. 17β-(tert.-Butyldimethylsilyloxy)-3-oxo-2'H,5'H-thieno[3',4':5,10]-5β-estr-9(11)-en-2'ξ-carbonsäuremethylester

Zu einer Suspension von 1,38 g (46,1 mmol) Natriumhydrid (80% in Öl) in 92,5 ml Dimethylformamid tropft man unter Eiswasserkühlung innerhalb von 3 min 2,92 ml (32,2 mmol) Mercaptoessigsäuremethylester und rührt weitere 15 min. Danach gibt man tropfenweise eine Lösung von 8,95 g (17,7 mmol) 17β-(tert.-Butyldimethylsilyloxy)-19-iod-androsta-4,9(11)-dien-3-on in 111 ml DMF hinzu und rührt 3 h bei Raumtemperatur. Zur Aufarbeitung gießt man in eiskalte gesättigte NH₄-Cl-Lösung und extrahiert mit Ethylacetat. Nach Chromatographie des Rohprodukts an Kieselgel mit Hexan/Ethylacetat 3:1 erhält man 7,08 g (79,2% d. Th.) der Titelverbindung als Isomerengemisch an C-2'. ¹H-NMR (CDCl₃, 300 MHz, 2'(R)-lsomeres): δ = 0,62 ppm (s,3H,H-18); 0,89 (s,9H,Si-tBu); 3,42 u. 3,68 (2d,J = 10 Hz,je 1H,H-19); 3,70 (t,J = 9 Hz,1H,H-17); 3,75 (s,3H,COOMe); 4,01 (s, 1H, H-2'); 5,83 (d,J = 7,5 Hz,1H,H-11).

### Beispiel D: 5-[2-(2-Pyrimidylsulfanyl)-ethyt]-5β-androst-9(11)-en-3β,17β-diol (16)

### a. 17β-(tert.-Butyldimethylsilyloxy)-3β-hydroxy-2'H,5'H-thieno[3',4':5,10]-5β-estr-9(11)-en-2'ξ-carbonsäuremethy)ester (10)

Zu einer Lösung von 3,20 g (6,34 mmol) des unter Beispiel 3b erhaltenen Isomerengemisches in 69 ml THF gibt man portionsweise unter Eiswasserkühlung 4,36 g (17,1 mmol) Lithium-tri-tert.-butoxyaluminiumhydrid. Nach Zugabe rührt man 3 h bei Raumtemperatur, zersetzt überschüssiges Reduktionsmittel durch vorschtige Zugabe von ca. 15 ml Wasser, filtriert über Celite, gießt das Filtrat in ca. 300 ml 5-proz. wäßrige Ammoniumchloridlösung und extrahiert mit Ethylacetat. Das Rohprodukt wird über Kieselgel mit Hexan/Ethylacetat 2:1 chromatographiert und liefert 2,35 g (73,1% d. Th.) des Reduktionsprodukts.

### b. 3β,17β-Bis-(tert.-butyldimethylsilyloxy)-2'H,5'H-thieno[3',4':5,10]-5β-estr-9(11)-en-2'ξ-carbonsäuremethylester (11)

Aus 2,35 g (4,64 mmol) des unter Da erhaltenen Reduktionsprodukts, 2,41 g (35,4 mmol) Imidazol und 3,14 ml (10,4 mmol) einer 3,3M Hexanlösung von tert.-Butyldimethylchlorsilan in 10,2 ml DMF erhält man unter den Bedingungen des Beispiels Ba 2,67 g (92,6% d. Th.) des Silylethers.

### c. 3β,17β-Bis-(tert.-butyldimethylsilyloxy)-2'H,5'H-thieno[3',4':5,10]-5β-estr-9(11)-en-2'ξ-methanol (12)

Zu einer Suspension von 193 mg (5,09 mmol) Lithiumaluminiumhydrid in 5,4 ml THF tropft man unter Eiswasserkühlung eine Lösung von 2,67 g (4,50 mmol) des unter Beispiel Db erhaltenen Produkts in 12,3 ml THF. Man rührt 2,5 h unter Eiswasserkühlung, zersetzt dann überschüssiges LiAIH₄ durch vorsichtige Zugabe von 2 ml Wasser, rührt weitere 30 min bei Raumtemperatur, filtriert über Celite, wäscht den Filterrückstand mit THF und Ethylacetat, nimmt das Filtrat in ca. 300 ml Wasser auf und trennt die Ethylacetatphase ab. Nach gründlicher Nachextraktion der wäßrigen Phase mit Ethylacetat, vereinigt man die EE-Extrakte, trocknet über Na₂SO₄ und engt ein. Das so erhaltene Rohprodukt (2,36 g, 100% d. Th.) wird ohne weitere Reinigung in die Folgestufe eingesetzt.
Zu analytischen Zwecken wird eine Probe des Rohprodukts aus Ethanol umkristallisiert. Auf diese Weise kann das 2'(R)-lsomere rein erhalten werden. Smp. 177° C, [α]_{D} +16,3° (CHCl₃, C = 0,516). ¹H-NMR (CDCl₃, 300 MHz): δ = 0,62 ppm (s,3H,H-18); 0,88 u. 0,90 (2s,je 9H,Si-tBu); 2,25 u. 3,22 (2d,J = 10 Hz,je 1H,H-19); 3,65 (t,J = 9 Hz,1H,H-17); 3,70 u. 3,87 (2m,je 1H,CH₂OH); 4,06 (s(br),1H,H-3); 4,95 (q,J = 6 u. 2 Hz,1H,H-2'); 5,59 (d,J = 7,5 Hz,1H,H-11).

### d. 3β,17β-Bis-(tert.-butyldimethylsilyloxy)-5-(2-hydroxyethyl)-5β-androst-9(11)-en (13)

Eine Suspension von 2,36 g (3,98 mmol) des voranstehend erhaltenen Produkts und 10 g Raney-Nickel in 92 ml Ethanol wird 3 h unter Rückfluß erhitzt. Nach dem Abkühlen wird die Reaktionslösung mit 100 ml Dichlormethan versetzt und über Celite filtriert. Der Filterrückstand wird gründlich mit Dichlormethan gewaschen. Nach dem Einengen des Filtrats verbleibt ein Rohprodukt von 2,36 g, das über Kieselgel mit Hexan/Ethylacetat chromatographiert wird. Die Hauptfraktion liefert 1,88 g (83,9% d. Th.) des kristallinen Entschwefelungsprodukts.

### e. 3β,17β-Bis-(tert.-butyldimethylsilyloxy)-5-(2-iodethyl)-5β-androst-9(11)-en (14)

Unter den Bedingungen des Beispiels Ac erhält man aus 1,88 g (3,34 mmol) des unter Dd gebildeten Alkohols, 2,24 g (8,53 mmol), 580 mg (8,53 mmol) Imidazol und 1,09 g (4,29 mmol) lod in 24,3 ml THF nach analoger Durchführung und Aufarbeitung 1,85 g (82,3% d. Th.) des lodids. ¹H-NMR (CDCl₃, 300 MHz): δ = 0,64 ppm (s,3H, H-18); 0,88 u. 0,93 (2s,je 9H,Si-tBu); 0,99 (s,3H,H-19); 3,20 u. 3,42 (2m,je 1H,CH₂l); 3,63 (t,J = 9 Hz,1 H,H-17); 4,05 (s(br),1H,H-3); 5,42 (d,J = 7,5 Hz,1H,H-11).

### f. 3β,17β-Bis-(tert.-butyldimethylsilyloxy)-5-[2-(2-pyrimidylsulfanyl)-ethyl]-5β-androst-9(11)-en (15)

Eine Suspension von 111 mg (2,54 mmol) NaH (55% in Öl) in 5 ml DMF wird nach Zugabe von 199 mg (1,77 mmol) Pyrimidin-2-thiol 15 min bei Raumtemperatur gerührt und anschließend tropfenweise mit einer Lösung von 655 mg (0,97 mmol) des voranstehend erhaltenen lodids in 6 ml THF und 6 ml Diethylether versetzt. Man rührt 21 h bei Umgebungstemperatur, gießt in eiskalte, gesättigte NaCl-Lösung und extrahiert mit Ethylacetat. Nach Chromatographie über Kieselgel mit Hexan/Ethylacetat 9:1 erhält man 600 mg (93,8% d. Th.) des kristallinen Substitutionsprodukts.

### g. 5-[2-(2-Pyrimidylsulfanyl)-ethyl]-5β-androst-9(11)-en-3β,17β-diol (16)

Eine Lösung von 590 mg (0,89 mmol) des unter Beispiel Df erhaltenen Produkts in 26,1 ml THF wird nach Zugabe von 2,76 g (8,74 mmol) Tetrabutylammoniumfluorid (Bu₄NF · 3 H₂O) 8 h bei 60° C gerührt. Nach dem Abkühlen gießt man in gesättigte NaHCO₃-Lösung und extrahiert mit Ethylacetat. Nach Chromatographie über Kieselgel mit Hexan/Ethylacetat 4:1 und Umkristallisation des Hauptprodukts aus Ethanol/Diisopropylether erhält man 250 mg (67,5% d. Th.) der Titelverbindung vom Smp. 209° C, [α]_{D} -27,4° (MeOH, C = 0,507). ¹H-NMR (DMSO-d₆, 300 MHz): δ = 0,59 ppm (s,3H,H-18); 0,93 (s,3H,H-19); 3,56 (m,1H,H-17); 3,93 (s(br),1H,OH); 4,30 (s(br),1H,H-3); 4,40 (m,1H,OH); 5,46 (d,J = 7,5 Hz,1H,H-11); 7,17 (t,J = 5 Hz,1H,H-5'); 8,60 (d,J = 5 Hz,2H,H-4' u. H-6').

Bei Verwendung der entsprechenden Thiole werden nach dem Verfahren des Beispiels D weitere Ausgangsverbindungen erhalten:
**1. 5-[2-(Heptylsulfanyl)-ethyl]-5β-androst-9(11)-en-3β,17β-diol,** Schmp. 126° C (Hexan/Ethylacetat), [α]_{D} +15,0° (CHCl₃, c = 0,453).
**2**. **5-[2-[(1-Methyl-1*H*-imidazol-2-yl)-sulfanyl]ethyl]-5β-androst-9(11)-en-3β,17β-diol,** Schmp. 212°C (Hexan/Ethylacetat), [α]_{D} +85,5° (CHCl₃, c = 0,503). ¹H-NMR (CDCl₃, 300 MHz): δ = 0,69 ppm (s,3H,H-18); 1,01 (s,3H,H-19); 3,55 (s,3H,Nme); 3,74 (t,J = 8 Hz,1H,H-17); 5,48 (m,1H,H-11); 6,87 (d,J = 0,5 Hz,1H,imidazol-H); 7,00 (d,J = 0,5 Hz,1H,imidazol-H).
**3. 5-[2-(Benzothiazol-2-yl)-sulfanyl]-5β-androst-9(11)-en-3β,17β-diol,** [α]_{D} +99,0° (CHCl₃, c = 0,5). ¹H-NMR (CDCl₃, 300 MHz): δ = 0,69 ppm (s,3H,H-18); 1,01 (s,3H,H-19); 4,18 (s,1H,H-3); 5,50 (m,1H,H-11); 7,27 (dd,J = 7,5 u. 8 Hz,1H,arom.-H); 7,41 (dd,J = 7,5 u. 8 Hz,1H,arom.-H); 7,73 (d,J 0 7,5 Hz,1H,arom.-H); 7,88 (d,J = 7,5 Hz,1H,arom.-H).
4. **5-[2-(Thien-2-yl)-sulfanyl]ethyl-5β-androst-9(11)-en-3β,17β-diol,** Schmp. 156° C [α]_{D} +3,0° (CHCl₃, c = 0,47). ¹H-NMR (CDCl₃, 300 MHz): δ = 0,65 ppm (s,3H,H-18); 0,99 (s,3H,H-19); 2,75 (m,1H,CH₂S); 3,08 (m,1H,CH₂S); 3,72 (t,J = 8 Hz,1H,H-17); 4,06 (s(br),1H,H-3); 5,45 (m,1H,H-11); 6,95 (dd,J = 4 u. 7 Hz,1H,thienyl-H); 7,12 (dd,J = 1 u. 4 Hz,1H,thienyl-H); 7,32 (dd,J 0 1 u. 7 Hz,1H,thienyl-H).

### Herstellung von Verbindungen der allgemeinen Formel II:

### Beispiel 1: 5-Ethyl-5β-androst-9(11)-en-3β,17β-diol

Eine Lösung von 2,25 g (3,34 mmol) 3β,17β-Bis-(tert.-butyldimethylsilyloxy)-5-(2-iodethyl-5β-androst-9(11)-en (Beispiel De) wird nach Zugabe von 50 mg Azobisiso-butyronitril auf 80° C erwärmt und tropfenweise mit 2 ml Tributylzinnhydrid versetzt. Man rührt weitere 60 min bei 80° C und gießt nach dem Abkühlen in 150 ml einer 5-proz. wäßrigen Natriumfluorid-Lösung. Das nach Extraktion mit Ethylacetat erhaltene Rohprodukt wird unter den Bedingungen des Beispiels Dg mit Tetrabutylammoniumfluorid in THF behandelt. Nach Chromatographie erhält man 720 mg (67,8%) der Titelverbindung vom Schmp. 165° C (Hexan/Ethylacetat), [α]_{D} +18,8 (CHCl₃, c = 0,493).

### Beispiel 2: 3β,17β-Dihydroxy-5β-androst-9(11)-en-5-propanitril

### a. 3β,17β-Bis-(tert.-butyldimethylsilyloxy)-5β-androst-9(11)en-5-propanitril

Eine Suspension von 2,23 g (3,31 mmol) 3β,17β-Bis-(tert.-butyldimethylsilyloxy)-5-(2-iodethyl)-5β-androst-9(11)-en (Beispiel De) und 948 mg (15,11 mmol) KCN in 48 ml DMF wird 36 Std. bei 60° C unter Argon gerührt. Nach dem Abkühlen gießt man in eiskalte 1 N NaOH-Lösung und extrahiert mit Dichlormethan. Nach Chromatographie des Rohprodukts über Kieselgel mit Hexan/Ethylacetat erhält man 1,56 g (75,5% d.Th.) des Nitrils vom Schmp. 194-195° C (Hexan), [α]_{D} +15,0° (CHCl₃, c = 0,5).

### b. 3β,17β-Dihydroxy-5β-androst-9(11)-en-5-propanitril

Eine Lösung von 300 mg (0,52 mmol) des voranstehenden Nitrils in 30 ml THF wird nach Zugabe von 2,67 g (10,3 mmol) Tetrabutylammoniumfluorid 2 Std. bei 60° C gerührt und unter den Bedingungen des Beispiels Dg aufgearbeitet. Man erhält nach Kristallisation aus Hexan/Ethylacetat 140 mg (77,8%) vom Schmp. 203° C, [α]_{D} +20,9° (CHCl₃, c = 0,496). ¹H-NMR (CDCl₃, 300 MHz): δ = 0,69 ppm (s,3H,H-18); 1,01 (s,3H,H-19); 2,52-2,75 (m,2H,CH₂CN); 3,73 (t,J = 8 Hz,1H,H-17); 4,10 (s(br),1H,H-3); 5,48 (m,1H,H-11).

## Patentansprüche

1. Verbindungen der allgemeinen Formel II mit dem Rest R in der Bedeutung von: R = -(CH₂)ₙ-CH₂-R¹, -(CH₂)ₙ-CH₂-OR¹, -(CH₂)ₙ-CH₂-OCOR¹, -(CH₂)ₙ-CH₂-SR¹, -(CH₂)ₙ-CH₂-NR¹R², -(CH₂)ₙ-CHO, -(CH₂)ₙ-CN worin n die Werte von 0-5 annehmen kann und die Reste R¹ und R² unabhängig voneinander für Wasserstoff oder einen geradkettigen oder verzweigten, gesättigten oder ungesättigten Kohlenwasserstoffrest mit bis zu 18 C-Atomen stehen, wobei dieser Rest gegebenenfalls zusätzliche funktionelle Gruppen und carbocyclische oder heterocyclische Ringelemente enthalten kann.

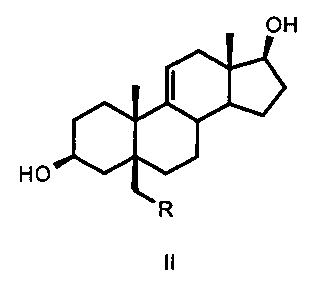

2. Verwendung der Verbindungen der allgemeinen Formel II gemäß Anspruch 1 zur Herstellung eines Arzneimittels zur Therapie Androgen-abhängiger Erkrankungen.

3. Pharmazeutische Mittel enthaltend mindestens ein Derivat der allgemeinen Formeln II und in der Galenik übliche, physiologisch verträgliche Hilfs- oder Trägerstoffe.

## Claims

1. Compounds of the general formula II where the R radical is defined as: R = -(CH₂)ₙ-CH₂-R¹, -(CH₂)ₙ-CH₂-OR¹, -(CH₂)ₙ-CH₂-OCOR¹, -(CH₂)ₙ-CH₂-SR¹, -(CH₂)ₙ-CH₂-NR¹R², -(CH₂)ₙ-CHO, -(CH₂)ₙ-CN, in which n may assume the values of 0-5 and the R¹ and R² radicals are each independently hydrogen or a straight-chain or branched, saturated or unsaturated hydrocarbon radical having up to 18 carbon atoms, where this radical may optionally contain additional functional groups and carbocyclic or heterocyclic ring elements.

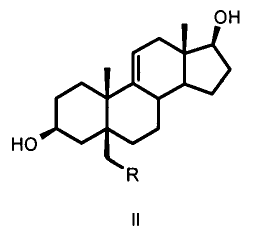

2. Use of the compounds of the general formula II according to Claim 1 for producing a medicament for the therapy of androgen-dependent disorders.

3. Pharmaceutical composition comprising at least one derivative of the general formula II and physiologically tolerated excipients or carriers customary in pharmaceuticals.

## Revendications

1. Composés de formule générale II dans laquelle le radical R signifie : R = -(CH₂)ₙ-CH₂-R¹, -(CH₂)ₙ-CH₂-OR¹, -(CH₂)ₙ-CH₂-OCOR¹, -(CH₂)ₙ-CH₂-SR¹, -(CH₂)ₙ-CH₂-NR¹R², -(CH₂)ₙ-CHO, -(CH₂)ₙ-CN dans laquelle n peut valoir 0-5 et les radicaux R¹ et R² représentent, indépendamment l'un de l'autre, hydrogène ou un radical hydrocarboné linéaire ou ramifié, saturé ou insaturé, comprenant jusqu'à 18 atomes de carbone, ce radical pouvant le cas échéant contenir des groupes fonctionnels supplémentaires et des éléments de cycle carboxyliques ou hétérocycliques.

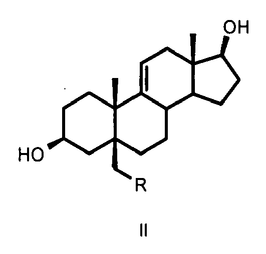

2. Utilisation des composés de formule générale Il selon la revendication 1 pour la préparation d'un médicament destiné à la thérapie de maladies dépendant des androgènes.

3. Préparation pharmaceutique, contenant au moins un dérivé de formule générale II et des adjuvants ou des substances support usuels en galénique, physiologiquement acceptables.
